# DEMANDE DE BREVET EUROPEEN

(11) **EP 3 449 949 A1**
(43) Date de publication de la demande: **06.03.2019**
(21) Numéro de dépôt: 18199444.3
(22) Date de dépôt: 22.07.2015
(51) Int. Cl.: A61L 2/10, H01J 61/00, A61L 9/20, A23L 3/28, A23B 7/015, A23L 5/30, A61L 2/26, G02B 5/08, H05B 41/39

(54) **DISPOSITIF UVC DE DÉCONTAMINATION ET DE DÉTOXIFICATION**

(30) Priorité: 25.07.2014 FR 1457205
(62) Demande divisionnaire de: 15748190.4
(71) Demandeur: Healthy Pulse, 91017 Evry Cedex (FR)
(72) Inventeur: SAFRAOUI, Georges, 91140 VILLEJUST (FR)
(74) Mandataire: Nony

(57) **Abrégé**

L'invention concerne un procédé de destruction d'agents pathogènes présents à la surface de fruits ou légumes, notamment de destruction de la patuline, comportant les étapes consistant à :
- entraîner en rotation de plus de 360° les objets à traiter,
- soumettre la surface des objets ainsi entraînés à une lumière riche en UVC émise par une ou plusieurs lampes flashs, la densité d'énergie du ou des flashs étant telle que la surface des fruits ou légumes soit exposée à une densité d'énergie d'au moins 1J/cm², à une densité de puissance d'au moins 2 kW/cm², et qu'au moins 20% de l'énergie reçue se trouve entre 200 et 315 nm.

## Description

La présente invention concerne les installations pour émettre des flashs riches en rayonnement UV, en vue de décontaminer ou de détoxifier des surfaces, par exemple la peau d'aliments tels que des fruits ou légumes. L'opération de détoxification vise à réduire notablement certaines mycotoxines telles que la patuline. La patuline est une molécule toxique résultat du métabolisme de certaines moisissures présentes sur les fruits et légumes.

Le rayonnement UV, et plus particulièrement UVC (180-280nm), présente une action biocide forte vis-à-vis d'agents potentiellement pathogènes ou indésirables tels que bactéries, virus, champignons ou levures, ... ou d'agents toxiques comme la patuline.

Il a ainsi été proposé d'utiliser des sources de rayonnement UV à base de lampes flashs.

Pour être efficaces, les flashs doivent véhiculer une énergie importante, ce qui pose un problème d'évacuation des calories générées et de tenue des matériaux utilisés.

De plus, les lampes flashs peuvent dans certaines situations ne pas émettre suffisamment, en raison par exemple d'un court-circuit accidentel qui fait que l'arc électrique est créé en dehors ou partiellement dans la lampe ; le rayonnement peut aussi être inadapté, en raison du vieillissement de la lampe qui tend à opacifier l'enveloppe en quartz de celle-ci. Actuellement, la mesure du courant généré vers la lampe ne permet de garantir ni qu'un flash a bien été émis, ni que la dose d'UVC nécessaire a été produite.

Il existe par ailleurs un besoin pour assurer la traçabilité des émissions d'UV, afin notamment de pouvoir garantir qu'un traitement de décontamination ou de détoxification a bien été effectué.

L'invention vise notamment à répondre à ce besoin et à perfectionner encore les installations de production de rayonnement UV.

Elle y parvient selon un premier de ses aspects grâce à un dispositif de décontamination par émission de flashs lumineux riches en rayonnement UV, notamment UVC, comportant :
- une lampe flash,
- un réflecteur, notamment disposé en arrière de la lampe flash, pour renvoyer la lumière émise par la lampe vers une fenêtre de sortie,
- un détecteur UV pour mesurer le rayonnement UV émis par la lampe.

La présence du détecteur UV permet de connaître le niveau de rayonnement UV effectivement émis. Ainsi, on peut d'une part vérifier que le flash a bien été produit, et d'autre part s'assurer que le niveau de production d'UV correspond à celui attendu.

Le réflecteur peut présenter un orifice et le détecteur UV peut être placé derrière cet orifice. Cela permet d'intégrer complètement le détecteur UV dans l'installation et d'associer un détecteur UV à chaque lampe flash utilisée. La détection des UV peut se faire au plus près de la lampe et ainsi fournir une image fiable du rayonnement émis.

Le diamètre de l'orifice est de préférence inférieur ou égal à 5 mm, mieux est compris entre 0,75 et 1,25mm, étant par exemple de 1mm.

Une grande partie des applications industrielles nécessitent une étanchéité de la tête optique. Dans ce cas, la fenêtre de sortie peut comporter une vitre transparente aux UV, en quartz synthétique de préférence, et assemblée par collage de préférence, à sa périphérie à un cadre de support. La surveillance du bris de cette vitre devient dans grand nombre d'applications alimentaires un élément de sécurité majeur.

De préférence, la vitre est revêtue à sa périphérie d'une première piste métallisée s'étendant le long d'au moins un grand côté de la vitre.

Cette première piste peut s'étendre selon une boucle ouverte, dont les extrémités se situent de préférence au niveau d'un petit côté de la vitre. La piste métallisée peut alors être reliée électriquement à un détecteur de continuité électrique, et être utilisée pour détecter une éventuelle fêlure de la vitre. En effet, une telle fêlure va interrompre la conduction électrique de la piste, ce qui pourra être détecté par le détecteur de continuité électrique. Afin d'assurer la rupture de cette piste conductrice, son épaisseur est de préférence inférieure à 100µm, mieux inférieure à 10µm, encore mieux inférieure à 1µm. La piste métallique peut être recouverte sur sa face externe d'un revêtement isolant électrique, notamment de silice, qui peut avoir été déposé sous vide. Cela réduit le risque de perturbation de la lecture de la conductivité de la piste. La piste est de préférence tournée du côté de la lampe flash.

Une deuxième piste, sans contrainte d'épaisseur, peut être déposée sur la vitre côté extérieur. Elle peut ainsi se superposer à un joint de colle assurant l'assemblage de la vitre et du cadre de support. Cette deuxième piste peut alors former écran vis-à-vis du rayonnement UV incident pour protéger ce joint de colle. Cela permet d'éviter un vieillissement prématuré du joint sous l'action des UVC.

Une solution avantageuse consiste à réunir au sein d'une même piste les deux fonctions, d'une part de surveillance de bris de la vitre et d'autre part de protection du joint de colle contre les UV émis par la lampe. La piste doit alors être suffisamment fine pour assurer la rupture dans le cas d'un bris de la vitre, et avoir préférentiellement une épaisseur de métal d'au moins 100nm afin de bloquer le rayonnement UV vers celui-ci, et être de préférence positionnée du coté du joint.

Le cadre de support peut être assemblé sur un boîtier contenant le réflecteur, avec interposition d'un joint d'étanchéité.

Le détecteur d'UV est de préférence porté par une carte électronique disposée au-dessus du réflecteur.

Le dispositif peut comporter un radiateur de support du réflecteur, présentant des gorges formées entre des ailettes et dans lesquelles sont reçus des tubes de circulation d'un liquide de refroidissement, notamment de l'eau.

Les tubes sont de préférence retenus par des pinces insérées entre les ailettes. Il est avantageux de ne pas coller ou fixer en dur les tubes au radiateur afin de permettre un léger déplacement, et le bon positionnement si nécessaire, de ceux-ci relativement au radiateur.

Le réflecteur est de préférence fixé sur le corps du radiateur avec interposition d'une feuille conductrice thermique. Cela permet de bien répartir la chaleur dégagée par la lampe sur toute la longueur du radiateur.

Les tubes peuvent être insérés à leurs extrémités dans des collecteurs munis de joints d'étanchéité, de préférence des joints toriques, s'appliquant sur les tubes.

Les collecteurs peuvent recevoir également les extrémités de la lampe flash.

La lampe flash peut être reçue dans une enveloppe en quartz, engagée dans les collecteurs.

Le dispositif comporte de préférence un circuit de contrôle mémorisant un historique d'émission d'UV des flashs, à partir du rayonnement détecté par le détecteur d'UV.

L'invention a encore pour objet, selon un autre de ses aspects, indépendamment ou en combinaison avec ce qui précède, un dispositif de production d'UV, comportant :
- une lampe flash,
- une vitre à travers laquelle les flashs sont émis, la vitre portant une piste conductrice formant une boucle ouverte reliée à un circuit électronique permettant de détecter un bris de la vitre par rupture de la continuité électrique de ladite piste.

L'invention a encore pour objet, selon un autre de ses aspects, indépendamment ou en combinaison avec ce qui précède, un dispositif de production d'UV, comportant :
- une lampe flash,
- une vitre à travers laquelle les flashs sont émis, la vitre étant collée par un joint de colle à un cadre de support, la vitre portant à sa périphérie une piste métallique se superposant au joint de colle et protégeant celui-ci du rayonnement UV émis par la lampe.

L'invention a encore pour objet, selon un autre de ses aspects, indépendamment ou en combinaison avec ce qui précède, un dispositif de production d'UV, comportant :
- une lampe flash,
- un réflecteur pour renvoyer la lumière émise par la lampe flash vers une fenêtre de sortie,
- un radiateur de support du réflecteur, le radiateur comportant un corps avec des ailettes et entre celles-ci des tubes dans lesquels circulent un liquide de refroidissement, les tubes étant plaqués contre le corps du radiateur de préférence par des pinces et reçus librement à leurs extrémités dans des collecteurs, avec interposition d'un joint torique entre le tube et le collecteur à ses extrémités.

Toutes les caractéristiques additionnelles présentées à l'occasion de la description du premier aspect de l'invention valent également pour ces autres aspects.

L'invention a encore pour objet, selon un autre de ses aspects, l'utilisation d'un dispositif selon l'invention pour détruire des agents pathogènes ou indésirables, tels que bactéries, virus, champignons ou levures, ou la destruction d'agents toxiques comme la patuline. On peut ainsi exposer au rayonnement UV des aliments, notamment des fruits ou légumes, avant par exemple de les traiter pour en faire une purée ou une compote.

Les aliments peuvent être déplacés en étant entrainés en rotation, sous l'installation.

On mesure avantageusement à chaque flash la dose de rayonnement UV émis par la lampe correspondante, à l'aide du détecteur UV précité. De préférence, on mémorise une information liée à l'émission UV de chaque flash ainsi que le nombre de flashs exécutés.

On peut modifier l'énergie envoyée dans la lampe en fonction du rayonnement émis par la lampe, mesuré précédemment, de façon à compenser la variation des caractéristiques d'émission en fonction du vieillissement de la lampe.

La continuité électrique de la piste précitée peut avantageusement être mesurée, de préférence avant et/ou après l'émission de chaque flash, afin de détecter l'état de la vitre.

De préférence, afin d'obtenir une réduction de toxine d'au moins un facteur 2, de préférence d'au moins 10, mieux supérieur à 100, on veille à ce que :
- l'on entraîne en rotation sur 360°, au minimum, l'objet à traiter, afin de traiter la totalité de la surface de cet objet ; la fréquence des flashs est telle que pendant la rotation minimum de 360° de l'objet à traiter, on ait au minimum 1 flash tous les 180° ou mieux un flash minimum tous les 120°, et/ou
- à une distance de 10cm, la tête optique délivre une fluence (ou densité d'énergie) en Joule/cm² comprise entre 1 et 3, et/ou
- à une distance de 10cm, la tête optique délivre une densité de puissance comprise entre 2kW/cm² et 15kW/cm², et/ou
- le flash de lumière émise est riche en UVC, c'est-à-dire présente une répartition spectrale telle qu'au moins 20% de l'énergie se trouve entre 200nm et 315nm

L'invention a ainsi encore pour objet un procédé de destruction d'agents pathogènes présents à la surface d'objets tels que des fruits ou légumes, notamment de destruction de la patuline, comportant les étapes consistant à :
- entraîner en rotation à 360°, au minimum, les objets à traiter,
- soumettre la surface des objets ainsi entraînés à la lumière riche en UVC émise par une ou plusieurs lampes flashs, la densité d'énergie du ou des flashs étant telle que la surface des objets soit exposée à une densité d'énergie d'au moins 1 J/cm², à une densité de puissance d'au moins 2 kW/cm², et qu'au moins 20% de l'énergie reçue se trouve entre 200 et 315nm.

Pour entraîner les objets en rotation, on peut amener les objets sous la ou les fenêtres d'émission de lumière grâce à un convoyeur comportant des rouleaux se déplaçant avec les objets et sur lesquels ceux-ci reposent, les rouleaux étant entraînés en rotation au moins lorsqu'ils passent sous lesdites fenêtres. En particulier, les rouleaux peuvent venir au contact d'une bande de friction qui les amène à rouler sur celle-ci. En variante, les rouleaux sont solidaires de roues dentées qui viennent en engagement avec une chaîne ou courroie crantée s'étendant sous la ou les fenêtres d'émission. Les objets traités peuvent être des pommes. La surface qui provoque la rotation des rouleaux peut être statique, auquel cas, c'est la vitesse de déplacement des rouleaux qui règle leur vitesse de rotation, ou mobile, ce qui permet de contrôler précisément la vitesse de rotation des rouleaux.

Les flashs peuvent provoquer la génération d'ozone à l'intérieur des enceintes contenant les lampes.

L'ozone ainsi généré peut dégrader certains matériaux à son contact. Par exemple, le réflecteur, lorsque réalisé en aluminium, peut s'oxyder et générer de l'alumine, sous la forme d'une poudre fine qui se dépose sur la vitre et occulte progressivement la lumière.

Avantageusement, on dispose dans chaque enceinte un absorbeur d'oxygène qui permet de purger l'air de l'oxygène et de réduire la formation d'ozone.

On installe ainsi dans l'enceinte, de préférence dans une zone non directement exposée à la lumière de la lampe flash, un contenant, par exemple sous forme de sachet, contenant une substance capable de réagir avec l'oxygène de l'air pour éliminer celui-ci de l'enceinte.

La substance est en une quantité suffisante pour transformer tout l'oxygène présent initialement dans le volume de l'enceinte, et pour maintenir un taux réduit d'oxygène dans l'enceinte pendant la durée recherchée, vu les petites fuites pouvant exister.

A chaque opération de maintenance, la substance est renouvelée.

La substance est par exemple sous forme pulvérulente, conditionnée en sachets.

La substance peut être à base de fer, notamment de carbonate de fer, qui réagit selon la réaction 4FeCO₃ + 6H₂O + O₂ → 4Fe(OH)₃ + 4CO₂.

L'avantage d'une telle substance est également d'absorber l'humidité de l'air, et de générer du CO₂ qui évite une mise en dépression de l'enceinte.

Il est également possible d'adjoindre un absorbeur d'humidité spécifique, tel qu'un gel de silice.

On peut utiliser un absorbeur d'oxygène non ferreux, tel qu'un ascorbate ou le bicarbonate de sodium.

Les sachets contenant la substance absorbeur d'oxygène peuvent être disposés dans un panier métallique ajouré, au contact de la substance contenue dans l'enceinte.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente, de façon schématique et partielle, en perspective, une installation de traitement selon l'invention,
- la figure 2 représente isolément, en perspective, un bloc optique de l'installation de la figure 1,
- la figure 3 représente en coupe longitudinale, partielle et schématique, le bloc optique de la figure 2,
- la figure 4 est une coupe transversale, partielle et schématique, du bloc optique,
- la figure 5 représente isolément la plaque de fermeture du bloc optique,
- la figure 6 illustre un détail du montage de la plaque de fermeture sur le boîtier du bloc optique,

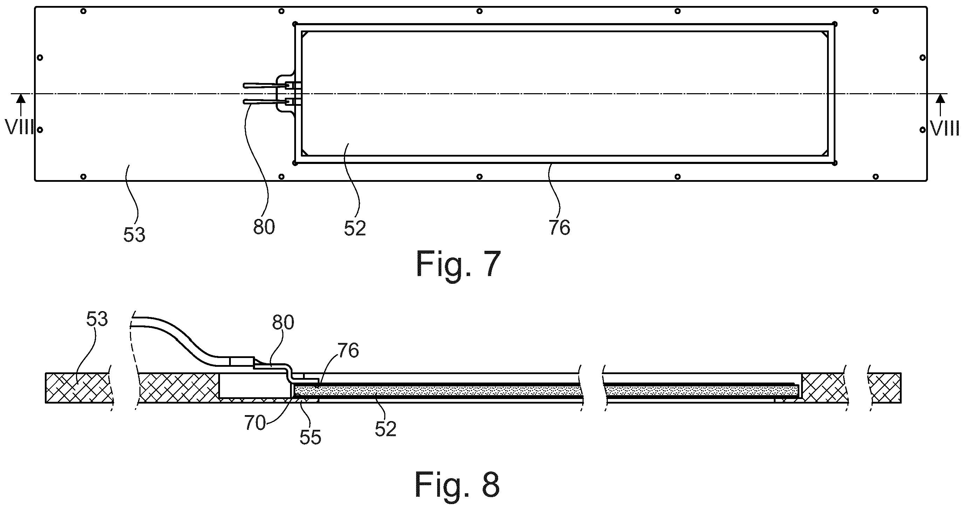

- la figure 7 représente en vue de dessus la plaque de fermeture,
- la figure 8 est une coupe longitudinale selon VIII-VIII de la figure 7,
- les figures 9 et 10 représentent des détails de réalisation du réflecteur, et
- les figures 11 et 12 illustrent de façon schématique deux exemples de convoyeurs pouvant être utilisés.

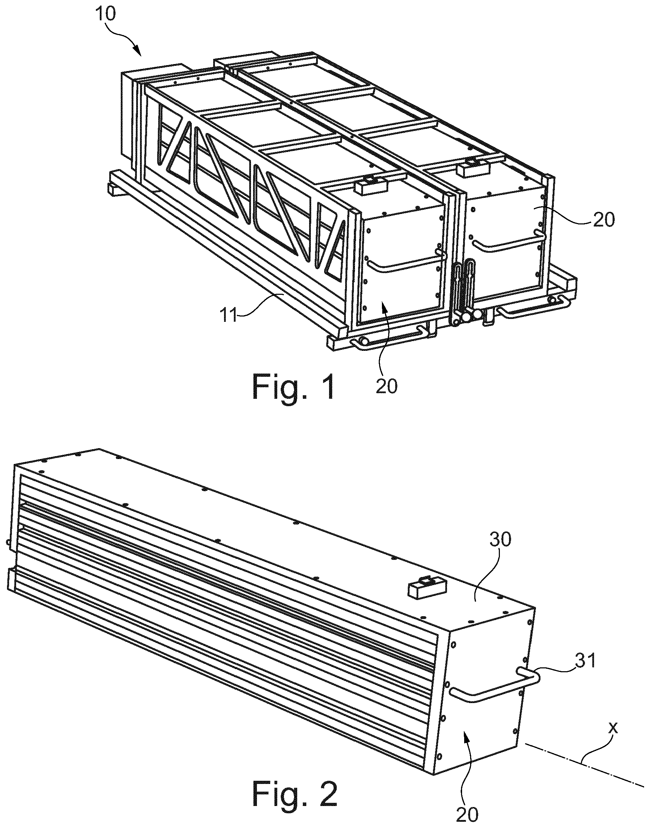

On a représenté partiellement à la figure 1 une installation 10 de décontamination selon l'invention, comportant un châssis 11 sur lequel sont fixés un ou plusieurs blocs optiques 20, au nombre de deux dans l'exemple illustré.

Des aliments ou autres produits à décontaminer sont déplacés sous les blocs optiques 20, à l'aide de tout convoyeur adapté. En variante, l'installation est agencée pour émettre le rayonnement UV vers une surface à décontaminer, qui est par exemple le sol ou le mur d'une pièce.

L'installation 10 comporte une alimentation électrique, non représentée, pour alimenter électriquement chaque bloc optique 20.

L'installation 10 comporte également des moyens de refroidissement par circulation d'un liquide, de préférence de l'eau.

Les blocs optiques 20 présentent avantageusement chacun, comme illustré sur la figure 2, une forme allongée selon un axe longitudinal X, et peuvent comporter chacun un boîtier 30 muni à l'avant d'une poignée 31 facilitant la mise en place sur et l'enlèvement du châssis 11.

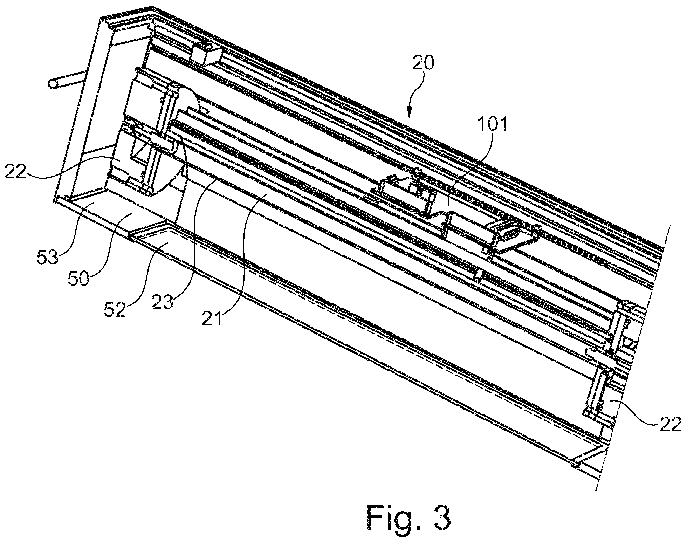

Si l'on se reporte à la figure 3, on voit que chaque bloc optique 20 comporte une lampe flash 21, de préférence rectiligne d'axe X, dont les extrémités sont reçues dans des collecteurs 22.

La lampe 21 s'étend à l'intérieur d'une enveloppe 23 sous forme de manchon en quartz, laquelle définit autour de la lampe un espace où peut circuler le liquide de refroidissement.

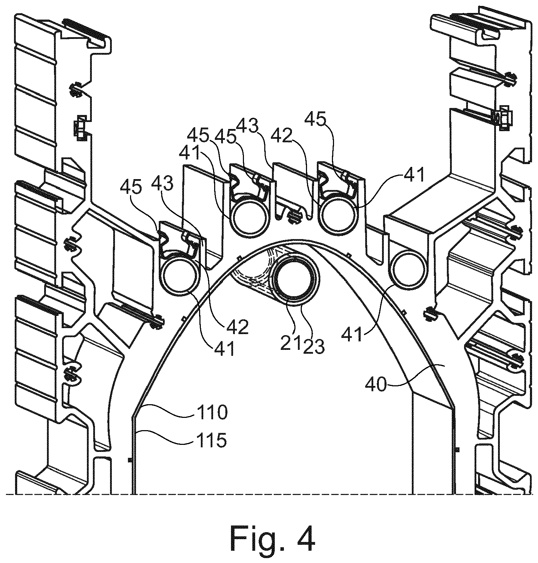

Comme on peut le voir plus particulièrement à la figure 4, le bloc 20 comporte un radiateur 40 sur lequel sont fixés des tubes 41, parcourus également par le liquide de refroidissement. Ce radiateur 40 supporte, du côté de la lampe, un réflecteur 110.

Le réflecteur 110 peut être formé d'une feuille en aluminium poli sur sa face tournée vers la lampe 21, et revêtue sur cette face d'une couche de quartz qui la protège de l'oxydation. Le polissage du réflecteur peut avoir été effectué électrolytiquement.

Une feuille 115 d'un matériau à haute conductivité thermique, de préférence un film chargé en céramique, de conductivité thermique supérieure ou égale à 2W/m.K, est interposée entre le réflecteur 110 et le radiateur 40.

Les tubes 41 sont reçus dans des gorges 42 formées entre des ailettes 43 du radiateur 40, et dont le fond de section semi-circulaire est adapté à leur diamètre.

De préférence, le maintien des tubes 41 dans le radiateur 40 s'effectue sans colle, ce qui facilite le montage et la maintenance.

En particulier, l'absence de colle autorise des tolérances de montage plus grandes au niveau de l'insertion des tubes 41 dans les collecteurs 22.

Une pâte thermiquement conductrice est de préférence disposée dans les gorges 42, pour améliorer la conduction thermique entre les tubes 41 et le radiateur 40.

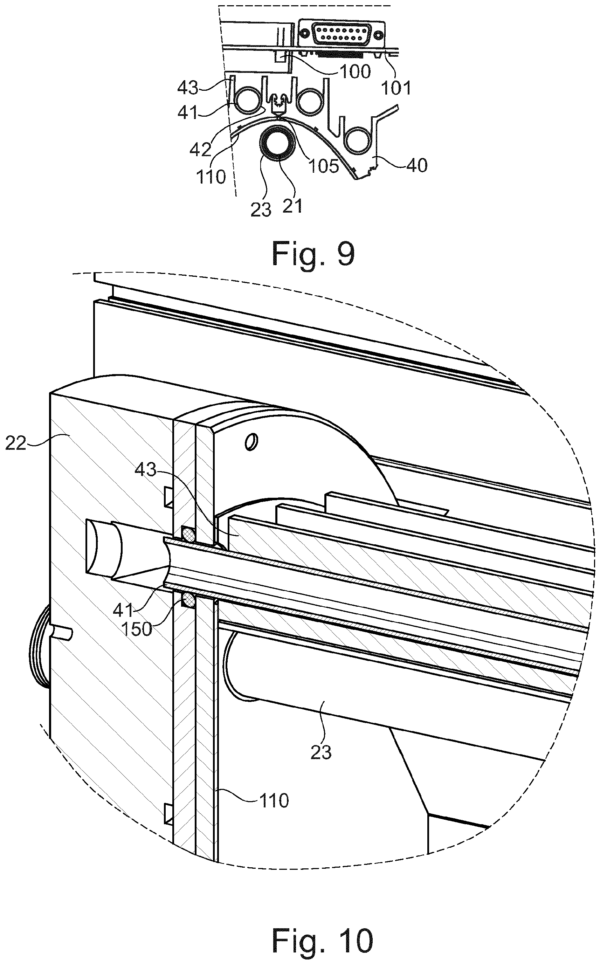

Les tubes 41 sont reçus, comme illustré à la figure 10, à leurs extrémités dans les collecteurs 22, qui assurent les interconnexions nécessaires à leur alimentation en liquide de refroidissement. Des joints toriques 150 peuvent s'appliquer à et effet sur les tubes 41.

Les tubes 41 peuvent être maintenus dans les gorges correspondantes 42 par des pinces 45 qui s'appuient sur les ailettes 43. Ces pinces 45 peuvent être mises en place après l'installation des tubes 41 dans les collecteurs 22.

En fonctionnement, le liquide de refroidissement circule en parallèle dans les tubes 41 et dans l'enveloppe 23.

Le boîtier 30 est fermé inférieurement par une plaque de fermeture 50.

Cette dernière comporte une vitre 52 et un cadre de support 53 en matériau opaque, par exemple en métal. La vitre 52 est de préférence en quartz synthétique, et peut faire entre 1,5 et 5mm d'épaisseur, 2mm par exemple.

Le cadre 53 définit une fenêtre délimitée par un rebord aminci 55, sur lequel la vitre 52 est fixée à l'aide d'un adhésif 56.

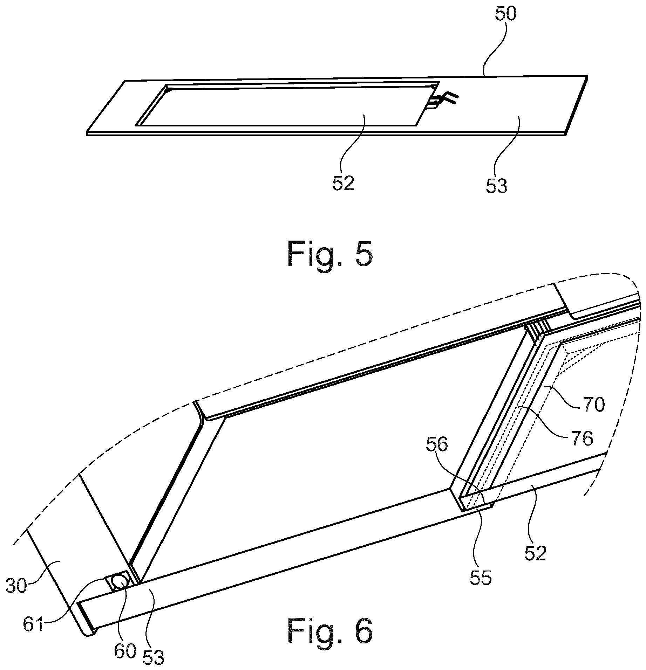

Un joint d'étanchéité 60 peut, comme illustré à la figure 6, être reçu dans une gorge 61 du boîtier 30 et s'appliquer sur le cadre 53 à sa périphérie.

De façon à protéger l'adhésif du rayonnement UV émis par la lampe flash, la vitre 52 porte une première piste métallique 70 qui forme écran vis-à-vis de ce rayonnement. La première piste 70 se situe sur la face de la vitre 52 tournée vers l'extérieur. Le métal de la première piste est de préférence de l'aluminium, et fait au moins 100 nm d'épaisseur, de préférence. La première piste présente une largeur qui est suffisamment grande pour protéger entièrement l'adhésif, par exemple quelques mm de large, notamment entre 4 et 6 mm de large, et s'étend sur toute la périphérie de la vitre. L'adhésif 56 s'étend entre la première piste 70 et le rebord 55.

La vitre 52 porte également, dans l'exemple illustré, une deuxième piste métallique qui forme une boucle ouverte à la périphérie de la face intérieure, tournée vers la lampe flash.

Des contacts 80 peuvent être soudés sur la deuxième piste 76. De préférence, celle-ci est revêtue, à l'exception de la zone de soudure des contacts, d'une couche d'un isolant électrique, par exemple de la silice, afin d'éviter que des salissures ou autre contact avec du métal qui viendraient à recouvrir la deuxième piste ne faussent la mesure de conductivité.

La deuxième piste 76 est par exemple moins large que la première, et fait par exemple 4,5 mm de large.

Dans l'exemple considéré, les première et deuxième pistes sont situées sur des faces opposées de la vitre 52, mais dans une variante les deux pistes peuvent être situées du même côté si la première est isolée électriquement de la deuxième.

En cas de fêlure de la vitre 52, la conduction de la deuxième piste 76 entre les contacts 80 est interrompue, ce qui peut être détecté électriquement par un circuit électronique adapté.

Il est alors possible d'interrompre l'émission des flashs et/ou de signaler l'anomalie.

Le bloc optique 20 comporte un détecteur 100 de rayonnement UV, monté sur un circuit imprimé 101, fixe relativement au radiateur 40.

Le détecteur 100 reçoit le rayonnement émis par la lampe 21 à travers un orifice 105 traversant le radiateur 40 et le réflecteur 110. L'orifice 105 fait par exemple 1 mm de diamètre.

La distance séparant l'entrée de l'orifice 105 côté lampe 21 et le détecteur 100, est par exemple comprise entre 1,5 et 2,5 cm.

Le détecteur 100 permet de connaître la quantité d'UVC émise à chaque flash et de vérifier que le bloc optique 20 émet bien la dose recherchée.

Le détecteur 100 est de préférence à base d'une photodiode de préférence en (Al)GaN (Aluminium-Gallium-Nitride) pour obtenir un gain significatif dans la bande des UVC.

L'installation 10 peut comporter un circuit électronique qui agit sur les paramètres d'alimentation de la lampe 21 afin de compenser l'usure de celle-ci. Par exemple, quand la lampe tend à s'obscurcir, on peut accroître l'intensité du courant afin d'émettre plus de rayonnement UV.

L'installation peut être agencée pour mémoriser la quantité d'UVC émise à chaque flash, de façon à permettre la détection d'une défaillance et autoriser une traçabilité de la décontamination effectuée.

L'installation peut comporter un système de nettoyage de la vitre 52, côté extérieur, par projection d'eau sous pression.

L'invention n'est pas limitée à l'exemple qui vient d'être décrit. En particulier, on peut modifier la forme du réflecteur ou du radiateur sans sortir du cadre de la présente invention.

L'invention s'applique avantageusement au traitement de fruits ou légumes, et notamment de pommes, en vue par exemple d'éliminer la patuline ou d'autres mycotoxines présentes à leur surface.

L'installation selon l'invention comporte avantageusement des moyens pour permettre de traiter l'ensemble de la surface des fruits ou légumes, en faisant effectuer à ceux-ci au moins une rotation sur eux-mêmes lors de leur passage sous les têtes de traitement qui émettent les flashs riches en UVC.

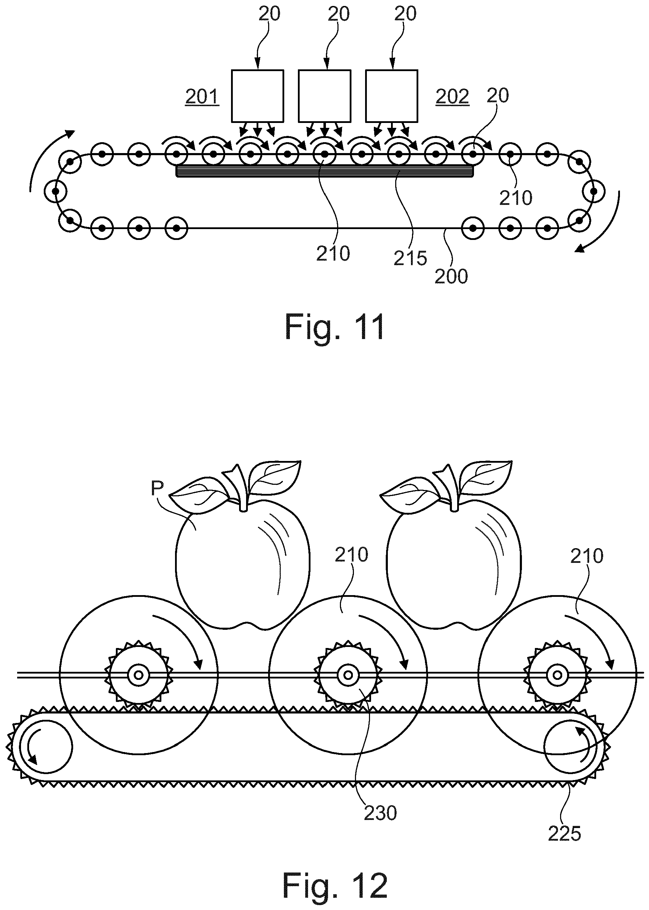

On a représenté à la figure 11 un premier exemple d'une telle installation. Celle-ci comporte un convoyeur 200 qui tourne en boucle fermée et passe devant les têtes de traitement 20, par exemple au nombre de trois. Les produits à traiter sont déposés en 201 en amont des têtes de traitement sur le convoyeur 200, et sont récupérés en aval de celles-ci, en 202.

Le convoyeur 200 comporte des rouleaux 210 sur lesquels reposent les produits à traiter.

Une bande de friction 215 s'étend au niveau des têtes de traitement ainsi qu'un peu en amont et en aval de celles-ci et les rouleaux 210 viennent à son contact. La bande de friction 215 amène les rouleaux 210 à tourner sur eux-mêmes au niveau des têtes de traitement, ce qui entraîne les produits en rotation. Le diamètre des rouleaux est choisi de façon à ce que les produits effectuent au moins une rotation sur eux-mêmes lors de leur passage sous les têtes de traitement 20, recevant ainsi plusieurs flashs riches en UVC, qui atteignent ensemble sensiblement toute leur surface.

Dans l'exemple illustré à la figure 11, la bande de friction 215 est fixe. On peut aussi la remplacer par une courroie entraînée en rotation dans le sens inverse de progression du convoyeur 200 de façon à entraîner les produits à traiter à la vitesse de rotation voulue, adaptée au traitement à effectuer.

Dans la variante de la figure 12, les rouleaux 210 sont solidaires chacun d'un pignon 230 et une chaîne ou courroie crantée 225 vient en prise avec chaque pignon 230 sous les têtes de traitement 20 pour entraîner le rouleau 210 correspondant à la vitesse de rotation souhaitée. De préférence, cette chaîne ou courroie crantée se déplace dans le sens inverse des rouleaux 210, ce qui permet d'accroître la vitesse relative entre les deux.

Avantageusement, comme indiqué précédemment, un absorbeur d'oxygène est disposé dans l'enceinte contenant la lampe.

L'expression « comportant un » doit se comprendre comme étant synonyme de « comprenant au moins un », sauf si le contraire est spécifié.

## Revendications

1. Procédé de destruction d'agents pathogènes présents à la surface de fruits ou légumes, notamment de destruction de la patuline, comportant les étapes consistant à :
- entraîner en rotation de plus de 360° les objets à traiter,
- soumettre la surface des objets ainsi entraînés à une lumière riche en UVC émise par une ou plusieurs lampes flashs, la densité d'énergie du ou des flashs étant telle que la surface des fruits ou légumes soit exposée à une densité d'énergie d'au moins 1J/cm², à une densité de puissance d'au moins 2 kW/cm², et qu'au moins 20% de l'énergie reçue se trouve entre 200 et 315 nm.

2. Procédé selon la revendication 1, les fruits ou légumes étant des pommes.

3. Procédé selon la revendication 1 ou 2, les objets reposant sur un convoyeur, le convoyeur comportant des rouleaux se déplaçant avec les objets, les rouleaux étant entraînés en rotation au moins lorsqu'ils passent sous des fenêtres d'émission de la lumière.

4. Procédé selon la revendication 3, les rouleaux venant au contact d'une bande de friction qui les amène à rouler sur celle-ci ou étant solidaires de roues dentées venant en engagement avec une courroie crantée ou une chaîne s'étendant sous la ou les fenêtres d'émission.

5. Procédé selon la revendication 3 ou 4, la surface provoquant la rotation des rouleaux étant statique, la vitesse de déplacement des rouleaux étant réglée par leur vitesse de rotation.

6. Procédé selon la revendication 3 ou 4, la surface provoquant la rotation des rouleaux étant mobile.

7. Procédé selon l'une quelconque des revendications précédentes, la ou les lampes flash étant disposées dans une enceinte et le procédé comportant une étape d'absorption d'oxygène par un absorbeur d'oxygène contenu dans l'enceinte pour réduire la formation d'ozone par la ou les lampes flash.
